# EUROPEAN PATENT APPLICATION

(11) **EP 3 799 058 A1**
(43) Date of publication of application: **31.03.2021**
(21) Application number: 19858105.0
(22) Date of filing: 03.09.2019
(51) Int. Cl.: G16H 10/60

(54) **MEDICAL RECORD SUMMARY INFORMATION GENERATING DEVICE, MEDICAL RECORD SUMMARY INFORMATION GENERATING METHOD, AND PROGRAM**

(30) Priority: 04.09.2018 JP 2018165027; 15.03.2019 JP 2019048413
(71) Applicant: Enishia Inc., Kyoto-shi, Kyoto 606-8501 (JP)
(72) Inventor: SAKAGUCHI, Tomohiro, Kyoto-shi, Kyoto 606-8501 (JP); ABE, Masakazu, Kyoto-shi, Kyoto 606-8501 (JP); YAMADA, Jin, Kyoto-shi, Kyoto 603-8161 (JP); OOE, Tadayuki, Kyoto-shi, Kyoto 604-8111 (JP); KOHIGASHI, Shigeo, Kyoto-shi, Kyoto 606-8501 (JP)
(74) Representative: Prüfer & Partner mbB Patentanwälte · Rechtsanwälte
(86) International application number: PCT/JP2019/034602
(87) International publication number: WO 2020/050266

(57) **Abstract**

A parameter DB (133) stores importance level information indicating an importance level of each element constituting a medical treatment record text indicated by medical treatment record information and being acquired by performing text analysis on the medical treatment record text, the importance level information being generated based on the element and a summary text relating to the medical treatment record text. An employed element estimator (114) estimates an element constituting a summary text, based on the importance level information and an element constituting a medical treatment record text indicated by new medical treatment record information and being acquired by performing text analysis on the new medical treatment record text. Further, the employed element estimator (114) generates new importance level information, based on a summary text after being corrected along a correction content received by a correction receiver (117) and an element constituting the new medical treatment record text.

## Description

### Technical Field

The present disclosure relates to a medical treatment record summary information generation device, a method of generating medical treatment record summary information, and a program.

### Background Art

Summary generation devices acquiring electronic medical record information including a medical treatment history of a patient, extracting a disease name from the acquired electronic medical record information, determining a disease type relating to the extracted disease name, and generating a summary of the electronic medical record information, based on the determined disease type have been proposed (see, for example, Patent Literature 1). The electronic medical record information is information in which a disease name acquired as a result of a diagnosis is summarized in a form of being associated with a date and a patient name.

### Citation List

### Patent Literature

Patent Literature 1: Unexamined Japanese Patent Application Publication No. 2009-157539

### Summary of Invention

### Technical Problem

However, electronic medical record information is not necessarily described in a form structured in consideration of subsequent extraction of a disease name and the like, and many pieces of electronic medical record information are described in an unstructured form such as text. Then, generation of suitable summary information from electronic medical record information described in a form such as text is requested. Generation of a summary provides advantages such as allowing a doctor to glance through medical treatment progress and efficiently recognize specifics of medical treatment, and facilitating transfer of medical treatment to another doctor. Further, generation of a summary also provides an advantage of facilitating generation of documents such as a medical treatment information providing document, a discharge summary, and a medical certificate.

The present disclosure has been made in view of the aforementioned ground, and an objective thereof is to provide a medical treatment record summary information generation device, a method of generating medical treatment record summary information, and a program that enable generation of suitable summary information from medical treatment record information.

### Solution to Problem

In order to achieve the aforementioned objective, a medical treatment record summary information generation device according to the present disclosure includes:
a medical treatment record storage storing medical treatment record information;
a summary storage storing summary information indicating a summary text relating to the medical treatment record information in association with the medical treatment record information;
an importance level storage storing importance level information indicating an importance level of each element constituting a medical treatment record text indicated by the medical treatment record information and being acquired by performing text analysis on the medical treatment record text, the importance level information being generated based on the element and a summary text relating to the medical treatment record text;
a medical treatment record receiver receiving new medical treatment record information;
an employed element estimator estimating an element constituting a summary text, based on the importance level information and an element constituting a medical treatment record text indicated by new medical treatment record information received by the medical treatment record receiver and being acquired by performing text analysis on the medical treatment record text;
a summary generator generating a summary text by use of an element estimated by the employed element estimator; and
a correction receiver receiving a correction content for the summary text by a user,
wherein the employed element estimator generates new importance level information, based on a summary text after being corrected along a correction content received by the correction receiver and an element constituting the medical treatment record text and updates importance level information stored by the importance level storage with generated new importance level information. A "user" corresponds to a doctor, another medical worker, or the like.

### Advantageous Effects of Invention

According to the present disclosure, the employed element estimator generates new importance level information, based on a summary text after being corrected along a correction content received by the correction receiver and an element constituting a medical treatment record text indicated by new medical treatment record information and being acquired by performing text analysis on the new medical treatment record text. Then, the employed element estimator updates importance level information stored by the importance level storage with the generated new importance level information. Consequently, the importance level information is updated to more suitable information, based on experiences of users (such as doctors and other medical workers), and therefore an advantage that validity of generated summary information is improved is provided. In other words, suitable summary information can be generated from medical treatment record information.

### Brief Description of Drawings

FIG. 1 is a block diagram illustrating a configuration of a medical treatment record summary information generation device according to an embodiment of the present disclosure;
FIG. 2A is a diagram illustrating contents of a medical treatment record database according to the embodiment;
FIG. 2B is a diagram illustrating contents of a summary database according to the embodiment;
FIG. 3A is a diagram illustrating contents of a parameter database according to the embodiment;
FIG. 3B is a diagram illustrating contents of an allowable range database according to the embodiment;
FIG. 4 is a flowchart illustrating an example of a flow of medical treatment record summary information generation processing according to the embodiment;
FIG. 5 is a flowchart illustrating an example of a flow of text analysis processing according to the embodiment;
FIG. 6 is a flowchart illustrating an example of a flow of element importance level setting processing according to the embodiment;
FIG. 7 is a flowchart illustrating an example of a flow of employed element estimation processing according to the embodiment;
FIG. 8 is a flowchart illustrating an example of a flow of medical treatment record summary information generation processing according to a modified example;
FIG. 9 is a flowchart illustrating an example of a flow of medical treatment record text constituent determination processing according to the modified example; and
FIG. 10 is a flowchart illustrating an example of a flow of medical treatment record summary information generation processing according to the modified example.

### Description of Embodiments

A medical treatment record summary information generation device according to an embodiment of the present disclosure will be described in detail below with reference to drawings. The medical treatment record summary information generation device according to the present embodiment includes a medical treatment record storage storing medical treatment record information, a summary storage storing summary information indicating a summary relating to a medical treatment record in association with the medical treatment record information, and an importance level storage. The importance level storage stores importance level information indicating an importance level of each element constituting a medical treatment record text indicated by medical treatment record information and being acquired by performing text analysis on the medical treatment record text, the importance level information being generated based on the element and a summary text relating to the medical treatment record text. The text analysis includes morphological analysis, syntactic analysis, contextual analysis, time information analysis, factuality analysis, named entity extraction, and semantic analysis. An "importance level" may also be determined for a combination of a plurality of elements. Further, the medical treatment record summary information generation device includes a medical treatment record receiver receiving new medical treatment record information, an employed element estimator estimating an element constituting a summary text, a summary generator generating summary information, a correction receiver receiving correction by a user on a summary text indicated by summary information, and a medical information analyzer. The employed element estimator estimates an element constituting a summary text, based on importance level information and an element constituting a medical treatment record text indicated by new medical treatment record information received by the medical treatment record receiver and being acquired by performing the text analysis on the new medical treatment record text. An "element" corresponds to at least one of a morpheme, a word, a phrase, a clause, a paragraph, and a sentence. Then, the summary generator generates summary information indicating a summary text by use of an element estimated by the employed element estimator. Further, the employed element estimator generates new importance level information, based on a summary text after being corrected along a correction content received by the correction receiver and an element constituting a medical treatment record text indicated by new medical treatment record information and being acquired by performing the text analysis on the new medical treatment record text and updates importance level information stored by the importance level storage with the generated new importance level information.

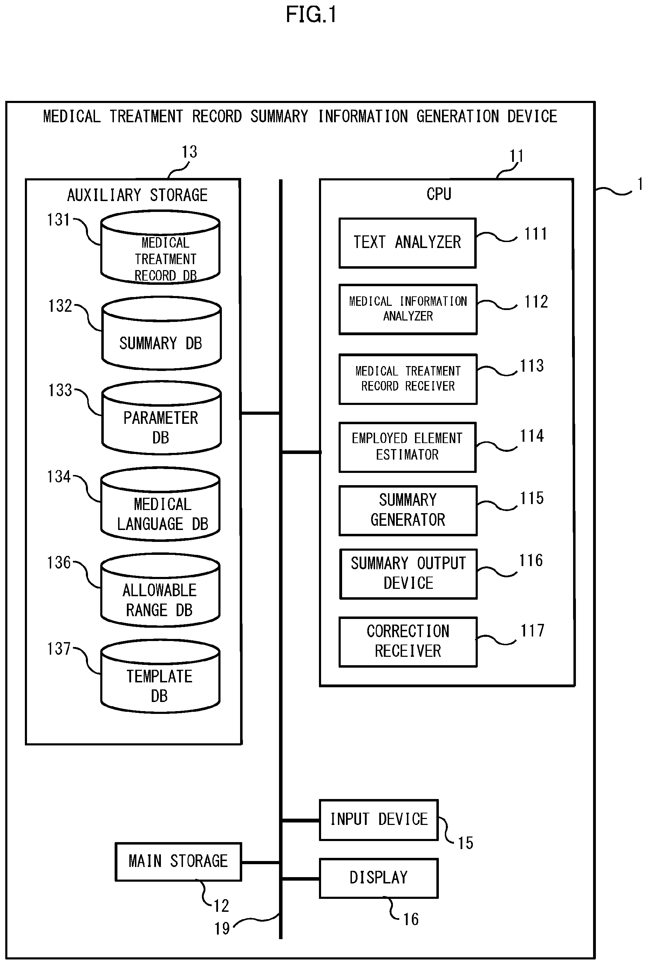

For example, as illustrated in FIG. 1, a medical treatment record summary information generation device 1 according to the present embodiment includes a central processing unit (CPU) 11, a main storage 12, an auxiliary storage 13, an input device 15, a display 16, and a bus 19 connecting the components. The main storage 12 is configured with a volatile memory such as a random access memory (RAM) and is used as a work area of the CPU 11. The auxiliary storage 13 is configured with a nonvolatile memory such as a magnetic disk or a semiconductor memory and stores a program for providing various functions of the medical treatment record summary information generation device 1. For example, the input device 15 is a keyboard, receives various types of operational information input by a user, and outputs the received operational information to the CPU 11. For example, the display 16 is a liquid crystal display and displays various types of information input from the CPU 11.

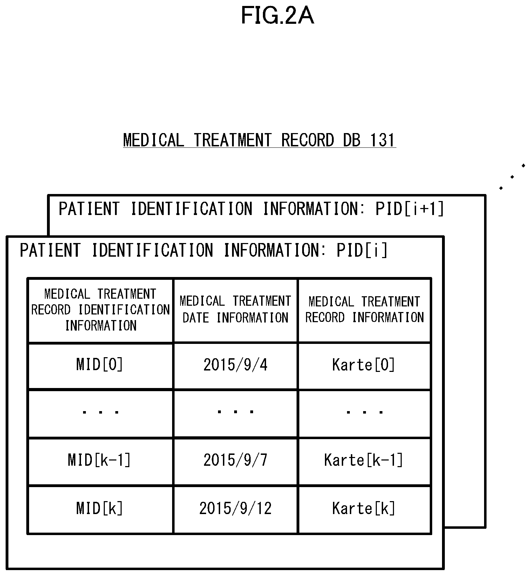

In the medical treatment record summary information generation device 1, the CPU 11 functions as a text analyzer 111, a medical information analyzer 112, a medical treatment record receiver 113, an employed element estimator 114, a summary generator 115, a summary output device 116, and a correction receiver 117 by reading a program stored by the auxiliary storage 13 into the main storage 12 and executing the program. Further, the auxiliary storage 13 includes a medical treatment record database (hereinafter referred to as "DB") 131, a summary DB 132, a parameter DB 133, a medical language DB 134, an allowable range DB 136, and a template DB 137. For example, the medical treatment record DB 131 stores medical treatment record information indicating past medical treatment record texts on a time-series basis for each patient, as illustrated in FIG. 2A. The medical treatment record DB 131 stores medical treatment record information in association with medical treatment record identification information, medical treatment date information, and progress identification information indicating progress of a medical treatment. Further, at least one medical treatment record information group for one patient is associated with patient identification information for identifying the one patient.

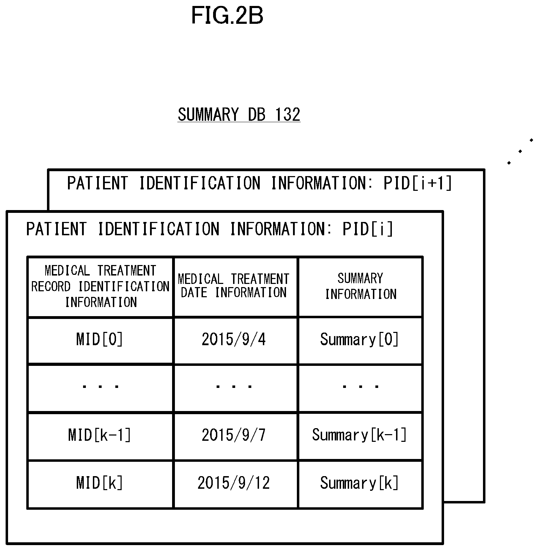

For example, the summary DB 132 stores summary information indicating past summary texts on a time-series basis for each patient, as illustrated in FIG. 2B. The summary DB 132 stores summary information in association with medical treatment record identification information for medical treatment record information relating to the summary information and medical treatment date information. Further, at least one summary information group for one patient is associated with patient identification information for identifying the one patient.

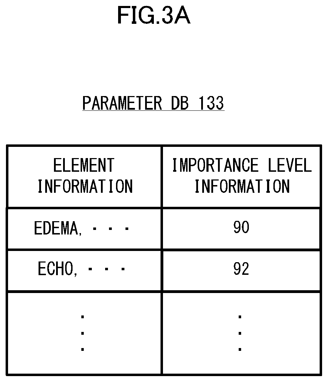

For example, the parameter DB 133 is an importance level storage storing importance level information indicating an importance level of each element, as illustrated in FIG. 3A. The importance level information is information indicating a degree to which an element is to be preferentially included in a summary text. Importance level information is generated based on an element constituting a medical treatment record text indicated by medical treatment record information and being acquired by performing the text analysis on the medical treatment record text and a summary text relating to the medical treatment record text.

Returning to FIG. 1, the medical language DB 134 is a medical linguistic information storage previously storing a plurality of types of medical languages generally used in medical treatment guidelines or other documents related to medical treatment.

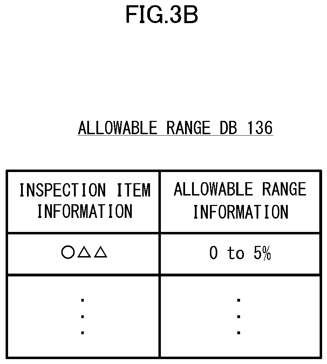

For example, the allowable range DB 136 is an allowable range storage storing allowable range information indicating an allowable range of an inspection value that may not need to be included in a summary text in terms of an inspection item defined by, for example, a medical treatment guideline, in association with medical linguistic information indicating the inspection item, as illustrated in FIG. 3B. For example, the example illustrated in FIG. 3B indicates that an allowable range of an inspection value relating to an inspection item "○ΔΔ" is 0 to 5%.

The template DB 137 stores summary template information for, when generating a summary text by use of an employed element, making the text a suitable summary text expression.

Returning to FIG. 1, the text analyzer 111 executes text analysis on a medical treatment record text indicated by medical treatment record information. The text analyzer 111 first divides the medical treatment record text indicated by the medical treatment record information into sentences. At this time, the text analyzer 111 divides the medical treatment record text into sentences, based on periods and the like included in the medical treatment record text. Next, by executing morphological analysis on each sentence acquired by dividing the medical treatment record text into sentences, the text analyzer 111 divides the sentence into elements. Then, by executing syntactic analysis on the sentence divided into elements, the text analyzer 111 estimates a structure of the sentence. Next, based on the estimated sentence structure, the text analyzer 111 estimates an anaphoric relation between words and phrases, a discourse relation between clauses, and the like. Further, by executing time information analysis on the sentence divided into elements, the text analyzer 111 estimates dates on which elements indicating events included in the text occur and the sequence of the events and arranges elements indicating the events on a time axis. Furthermore, by executing factuality analysis on the sentence divided into elements, the text analyzer 111 determines whether an event included in the text has actually occurred.

Referring to medical linguistic information stored by the medical language DB 134, the medical information analyzer 112 extracts an element relating to a medical-field-specific expression from elements constituting a medical treatment record text and being acquired by performing the text analysis on medical treatment record information. Specifically, the medical information analyzer 112 extracts elements relating to medical-field-specific expressions from a sentence divided into elements included in medical treatment record information and estimates a medical meaning of each extracted element by executing semantic analysis. With respect to each element relating to a medical expression, the medical information analyzer 112 estimates a superordinate concept or a subordinate concept of the element and estimates whether the element is a synonym or a polyseme. Further, the medical information analyzer 112 determines whether an inspection value included in a medical treatment record text indicated by medical treatment record information falls within an allowable range indicated by allowable range information relating to the inspection value, the range being stored in the allowable range DB 136.

The medical treatment record receiver 113 receives new medical treatment record information input by a user through the input device 15.

The employed element estimator 114 estimates an element constituting a summary text, based on importance level information stored by the parameter DB 133 and an element constituting a medical treatment record text indicated by new medical treatment record information received by the medical treatment record receiver 113 and being acquired by performing the text analysis on the medical treatment record text. Specifically, based on importance level information indicating an importance level of each element, the importance level information being stored by the parameter DB 133, the employed element estimator 114 determines importance level information of each element constituting a medical treatment record text indicated by new medical treatment record information received by the medical treatment record receiver 113 and being acquired by performing the text analysis on the new medical treatment record text. Then, the employed element estimator 114 estimates an element importance level information of which is equal to or greater than a preset reference value as an employed element.

Further, the employed element estimator 114 generates new importance level information, based on a summary text after being corrected along a correction content received by the correction receiver 117 and an element constituting a new medical treatment record text and being acquired by performing the text analysis on the new medical treatment record text. Then, the employed element estimator 114 updates the importance level information stored by the parameter DB 133 with the generated new importance level information. Subsequently, the employed element estimator 114 estimates an employed element by use of the newly generated importance level information. Thus, the employed element estimator 114 has a function of updating the importance level information by use of machine learning, based on a corrected summary text and new medical treatment record information.

The summary generator 115 generates summary information indicating a summary text by use of an employed element estimated by the employed element estimator 114, based on the summary template information stored by the template DB 137.

The summary output device 116 outputs summary information generated by the summary generator 115 to the display 16. At this time, the display 16 displays a summary text indicated by the summary information input from the summary output device 116. Consequently, referring to the summary text displayed on the display 16, a user can correct the summary text through the input device 15.

When a user performs an operation of correcting a summary text through the input device 15 in a state of the summary text being displayed on the display 16 by the summary output device 116, the correction receiver 117 receives a correction content for the summary text.

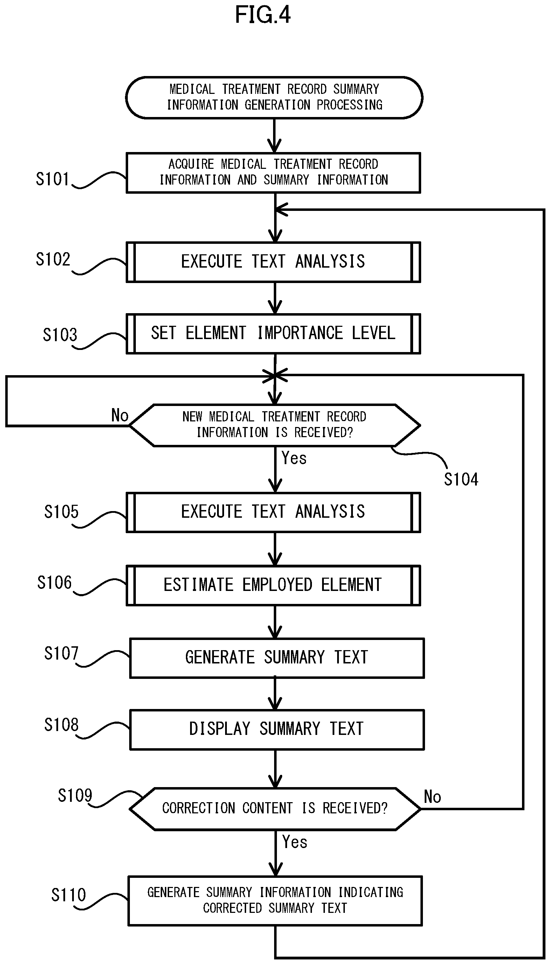

Next, medical treatment record summary information generation processing executed by the medical treatment record summary information generation device 1 according to the present embodiment will be described with reference to FIG. 4 to FIG. 7. The medical treatment record summary information generation processing is started with a user performing an operation for executing the medical treatment record summary information generation processing through the input device 15 after turning on the power to the medical treatment record summary information generation device 1 as a trigger.

First, the text analyzer 111 and the medical information analyzer 112 acquire past medical treatment record information and summary information therefor from the medical treatment record DB 131 and the summary DB 132, as described in FIG. 4 (Step S101).

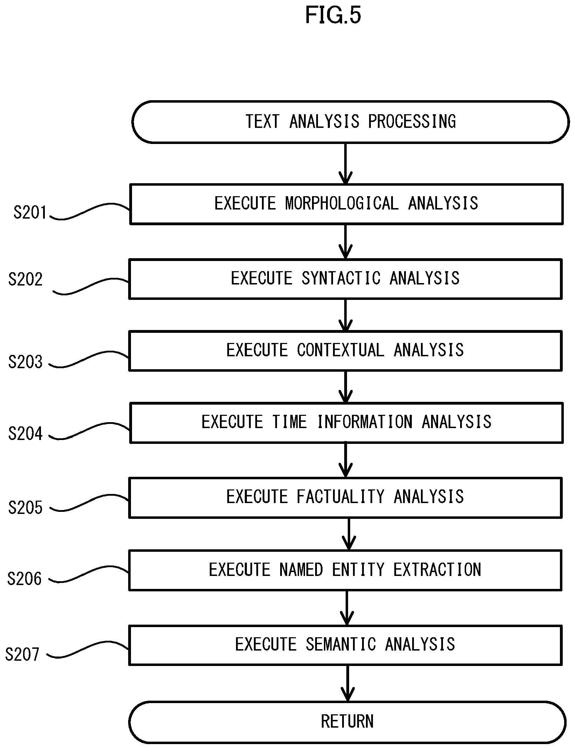

Next, the text analyzer 111 executes text analysis processing (Step S102). In the text analysis processing, first, referring to the medical linguistic information stored by the medical language DB 134, the text analyzer 111 divides a medical treatment record text indicated by the medical treatment record information and the summary text into elements by executing morphological analysis on the medical treatment record text, as described in FIG. 5 (Step S201). Next, the text analyzer 111 executes syntactic analysis on the medical treatment record text divided into elements (Step S202). Next, the text analyzer 111 executes contextual analysis on the medical treatment record text divided into elements (Step S203). Subsequently, the text analyzer 111 executes time information analysis on the medical treatment record text divided into elements (Step S204). Next, the text analyzer 111 executes factuality analysis on the medical treatment record text divided into elements (Step S205). Next, referring to the medical linguistic information stored by the medical language DB 134, the medical information analyzer 112 extracts a medical-field-specific expression from the sentence divided into elements (Step S206). Then, referring to the medical linguistic information stored by the medical language DB 134, the medical information analyzer 112 executes semantic analysis of estimating a medical meaning of each extracted element (Step S207).

Returning to FIG. 4, next, the employed element estimator 114 executes element importance level setting processing, based on the result of the text analysis executed by the text analyzer 111 on a medical treatment record text indicated by the past medical treatment record information (Step S103).

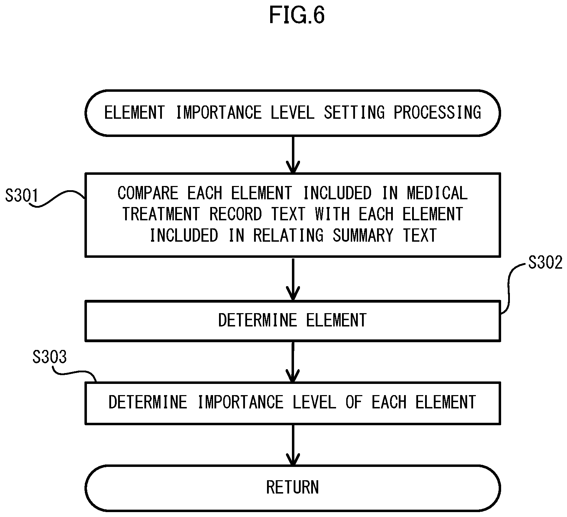

In the element importance level setting processing, first, based on the result of the text analysis executed by the text analyzer 111 on a medical treatment record text indicated by medical treatment record information, the employed element estimator 114 compares each element included in the medical treatment record text with each element included in a relating summary text, as described in FIG. 6 (Step S301).

Next, based on the result of comparing each element included in the medical treatment record text with each element included in the relating summary text, the employed element estimator 114 determines an element employed in the summary text (Step S302). Next, the employed element estimator 114 determines an importance level of each determined element by use of machine learning (Step S303). The employed element estimator 114 causes the parameter DB 133 to store importance level information indicating the determined importance level of each element.

In the determination of an importance level of each element by machine learning performed by the employed element estimator 114 (Step S303), the importance level is determined by use of linguistic information and medical information included in each element acquired in the text analysis processing (Step S102). For example, the linguistic information and the medical information include a disease name, a symptom, a date, a prescription medicine, whether a certain inspection value is a normal value, and a per-unit-period variation in a certain inspection value.

Next, the text analyzer 111 determines whether new medical treatment record information is received (Step S104). Unless new medical treatment record information is received (Step S104: No), the text analyzer 111 repeats the processing in Step S104.

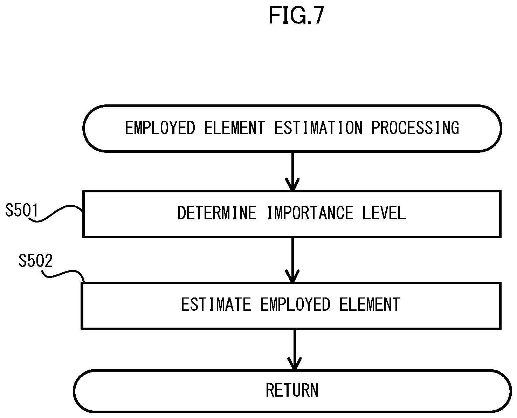

On the other hand, when determining that new medical treatment record information is received (Step S104: Yes), the text analyzer 111 executes the text analysis processing on the new medical treatment record information (Step S105). Then, the employed element estimator 114 executes employed element estimation processing, based on an element constituting a new medical treatment record text and being acquired by performing the text analysis on the new medical treatment record information (Step S106). In the employed element estimation processing, first, based on each element constituting the new medical treatment record text, the employed element estimator 114 determines an importance level of the element included in the new medical treatment record text, as described in FIG. 7 (Step S501). The employed element estimator 114 determines an importance level of each element included in the medical treatment record text, based on an importance level indicated by importance level information relating to each element, the importance level information being stored in the parameter DB 133. Next, the employed element estimator 114 estimates an element the determined importance level of which is equal to or greater than a preset reference importance level as an employed element (Step S502).

Returning to FIG. 4, next, the summary generator 115 generates a summary text by use of an employed element estimated by the employed element estimator 114, based on the summary template information stored by the template DB 137 (Step S107). The summary generator 115 causes the summary DB 132 to store the generated summary information. Subsequently, the summary output device 116 outputs the summary information generated by the summary generator 115 to the display 16 (Step S108). At this time, the display 16 displays a summary text indicated by the summary information input from the summary output device 116.

Next, the correction receiver 117 determines whether a correction content for the summary text is received in a state of the summary text being displayed on the display 16 by the summary output device 116 (Step S109). When a user performs an operation for acknowledging the content of the displayed summary text as-is through the input device 15 in a state of the summary text being displayed on the display 16 by the summary output device 116, the correction receiver 117 determines that a correction content for the summary text is not received. On the other hand, when a user performs an operation for acquiring a content of the displayed summary text through the input device 15 in a state of the summary text being displayed on the display 16 by the summary output device 116, the correction receiver 117 determines that a correction content for the summary text is received.

When the correction receiver 117 determines that a correction content for the summary text is not received (Step S109: No), the processing in Step S104 is executed again. On the other hand, when determining that a correction content for the summary text is received (Step S109: Yes) the correction receiver 117 notifies information indicating the correction content to the summary generator 115. Then, the summary generator 115 generates summary information indicating a corrected summary text, based on the information indicating the correction content (Step S110). Further, the summary generator 115 updates summary information relating to new medical treatment record information, the summary information being stored in the summary DB 132, by use of the summary information indicating the corrected summary text.

Subsequently, the text analyzer 111 executes the aforementioned text analysis processing again by use of the updated summary information (Step S102). Next, the element importance level setting processing in Step S103 is executed again. At this time, the employed element estimator 114 generates new importance level information, based on the summary text after undergoing the correction received by the correction receiver 117 and an element constituting a new medical treatment record text indicated by the new medical treatment record information and being acquired by performing the text analysis on the new medical treatment record text. Then, the employed element estimator 114 causes the parameter DB 133 to store the generated new importance level information. Next, the processing in and after Step S103 is executed. By thus repeating execution of the processing from Step S103 to Step S110, machine learning of importance level information used by the employed element estimator 114 is performed.

As described above, the medical information analyzer 112 generates new importance level information, based on a summary text after undergoing a correction received by the correction receiver 117 and an element constituting a medical treatment record text indicated by new medical treatment record information and being acquired by performing the text analysis on the new medical treatment record text, in the medical treatment record summary information generation device 1 according to the present embodiment. Then, the employed element estimator 114 updates the importance level information stored by the importance level storage with the generated new importance level information. Consequently, the importance level information stored by the parameter DB 133 is updated to more suitable information, based on experiences of doctors, other medical workers, and the like, and therefore an advantage that validity of generated summary information improves is provided.

Further, based on an element constituting a medical treatment record text indicated by medical treatment record information and being acquired by performing the text analysis on the medical treatment record text and a summary text relating to the medical treatment record text, the text analyzer 111 determines a medical language from the medical treatment record text and the summary text, referring to the medical linguistic information stored by the medical language DB 134, according to the present embodiment,. Consequently, the probability of a medical language stored by the medical language DB 134 being omitted from a summary text can be reduced, and therefore an advantage that summary information suitable for a user is generated by, for example, causing the medical language DB 134 to store a medical language important for the user is provided.

Further, for example, when an inspection value indicated by inspection value information included in medical treatment record information is out of an allowable range, the likelihood of the inspection value being included in a summary text increases, and therefore overlook of abnormality of an inspection value originating in a disease of a patient is suppressed, according to the present embodiment.

While the embodiment of the present disclosure has been described above, the present disclosure is not limited to the configuration of the aforementioned embodiment. For example, the medical information analyzer 112 may determine whether the absolute value of per-unit-period variation in an inspection value indicated by inspection value information included in past medical treatment record information and new medical treatment record information of a patient is out of an allowable range, based on a history of inspection values indicated by the inspection value information. In this case, the allowable range DB 136 may store allowable range information indicating a preset allowable range for the absolute value of a per-unit-period variation in an inspection value indicated by inspection value information included in the past medical treatment record information and the new medical treatment record information.

For example, based on an element constituting a medical treatment record text indicated by new medical treatment record information received by the medical treatment record receiver 113 and being acquired by performing the text analysis on past medical treatment record information stored by the medical treatment record DB 131 and the new medical treatment record text, the medical information analyzer 112 determines inspection value information included in each piece of the past medical treatment record information and the new medical treatment record information. Then, based on a history of an inspection value indicated by the determined inspection value information, the medical information analyzer 112 determines whether the absolute value of a per-unit-period variation in the inspection value indicated by the determined inspection value information is out of the aforementioned allowable range. When the medical information analyzer 112 determines that the absolute value of the per-unit-period variation is out of the aforementioned allowable range, the summary generator 115 may generate summary information in such a way that the inspection value indicated by the inspection value information is included in the summary text.

In this configuration, when a variation in an inspection value of a preset inspection item is out of a preset allowable range, the likelihood of the inspection item and the inspection value being included in a summary text increases. Accordingly, for example, a user can prevent omission of a variation in an inspection value originating in a disease of a patient.

For example, when generating a summary text at discharge of a patient (a so-called discharge summary), the medical treatment record summary information generation device according to the embodiment may select all summary texts generated during hospitalization of the patient and display the texts on the display 16. Thus generating a so-called discharge summary, a medical treatment information providing document, a medical certificate, and the like provides an advantage that efficiency of document generation by doctors and the like can be enhanced.

An example of the text analysis processing executed on a medical treatment record text by the text analyzer 111 and the medical information analyzer 112 including morphological analysis, syntactic analysis, contextual analysis, time information analysis, factuality analysis, named entity extraction, and semantic analysis has been described in the embodiment. However, the text analysis processing executed by the text analyzer 111 and the medical information analyzer 112 may include other types of analytical processing.

Importance level information according to the embodiment may include not only information indicating an importance level of one element but also information indicating an importance level of each combination of a plurality of elements.

An important element storage (unillustrated) storing important element information indicating a predetermined important element and an important element output device (unillustrated) outputting important element information to the display 16 may be included in the embodiment. The "predetermined important element" means an important element to be intrinsically included in a summary text whether the element is included in past medical treatment record information or not. In other words, the preset important element means medical treatment storage information, that is, a so-called "complementary important element" which is to be included in a so-called original in a generation stage of the original but is overlooked and is highly likely not to appear in the original. When the summary output device 116 outputs summary information to the display 16, the important element output device acquires the aforementioned important element information from the important element storage and outputs the acquired important element information to the display 16. Specifically, the important element output device outputs an important element that may not be estimated by the employed element estimator 114 or that may be overlooked, such as an inspection name, a treatment name, a prescription name, or a diagnosis name, to the display 16 along with summary information. In this case, the display 16 displays the important element indicated by the important element information input from the important element output device along with the summary text indicated by the summary information input from the summary output device 116. This configuration allows prevention of omission of a predetermined important element from a summary text, and therefore a more suitable summary text can be generated.

The text analyzer 111 according to the embodiment may estimate an element constituting a medical treatment record text by determining at least one of a method of punctuating a sentence in a summary text, a method of summary text structuring based on a date, a keyword, and the like, and a form of expressing the summary text. In this case, the employed element estimator 114 may set an importance level to each element constituting the medical treatment record text estimated based on at least one of a method of punctuating a sentence in the summary text, a method of summary text structuring, and a form of expressing the summary text.

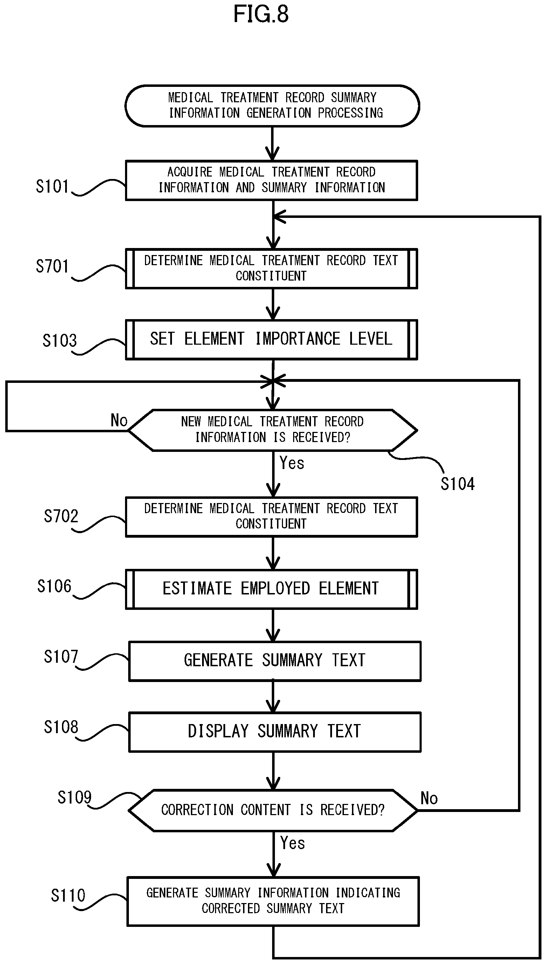

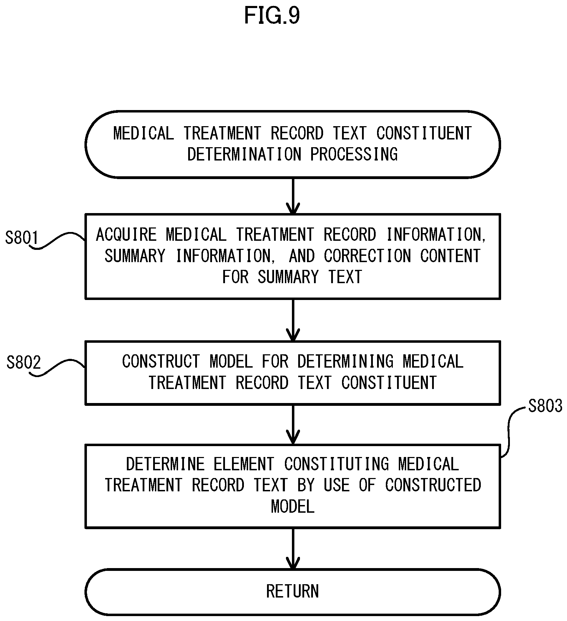

Medical treatment record summary information generation processing executed by a medical treatment record summary information generation device 1 according to this modified example will be described with reference to FIG. 8 and FIG. 9. In FIG. 8, processing similar to that in the embodiment is given the same sign as that in FIG. 4. For example, in the medical treatment record summary information generation processing according to this modified example, medical treatment record text constituent determination processing (Steps S701 and S702) is executed in place of the text analysis processing described in the embodiment, as described in FIG. 8. For example, a case in which only past medical treatment record information exists and summary information does not exist in Step S101 in FIG. 8 includes a case in which, for example, after a doctor causes the summary DB 132 to store summary information manually generated from several pieces of past medical treatment record information, the text analyzer 111 and the medical information analyzer 112 acquires the summary information from the summary DB 132. For example, in the medical treatment record text constituent determination processing, first, the text analyzer 111 acquires medical treatment record information, summary information, and a correction content for a summary text, as described in FIG. 9 (Step S801). When the correction receiver 117 does not receive a correction content for a summary text, the text analyzer 111 acquires only medical treatment record information and summary information.

Next, the text analyzer 111 constructs a model for determining a constituent of a medical treatment record text indicated by the medical treatment record information, based on a method of punctuating a sentence in a summary text, a method of summary text structuring, and a form of expressing the summary text (Step S802). When the correction receiver 117 receives a correction content, the summary text also includes a summary text after being corrected along the received correction content. Next, the text analyzer 111 determines an element constituting the medical treatment record text, based on the medical treatment record text and the constructed model (Step S803).

For example, this configuration can provide, at a hospital holding medical treatment record information, summary information reflecting a method of punctuating a sentence in a summary text unique to the hospital, a method of summary text structuring based on a date, keyword, and the like, or a form of expressing the summary text.

The correction receiver 117 according to the embodiment may receive a correction content including not only a mere correction related to selection of an element employed in a summary text but also a correction of a method of punctuating a sentence in the summary text, a method of extracting an element to be included in the summary text from medical treatment record information, the result of summary text structuring based on a date, a keyword, and the like, or an expression of the summary text.

An example of the text analyzer 111 and the medical information analyzer 112 executing the text analysis processing and the element importance level setting processing by use of past medical treatment record information and summary information therefor every time a correction content for a summary text generated from new medical treatment record information is received after the new medical treatment record information is received has been described in the embodiment. However, without being limited to the above, for example, the text analyzer 111 and the medical information analyzer 112 may accumulate a correction content received by the correction receiver 117 until a preset element importance level update time arrives and execute the text analysis processing and the element importance level setting processing by use of past medical treatment record information and summary information therefor when the element importance level update time arrives. Specifically, the text analyzer 111 and the medical information analyzer 112 may perform so-called batch processing of accumulating a correction content received by the correction receiver 117 in a period up to the element importance level update time and updating an element importance level by use of the correction content accumulated by then at a timing when the element importance level update time arrives. For example, the element importance level update time may arrive every day, every week, or every month.

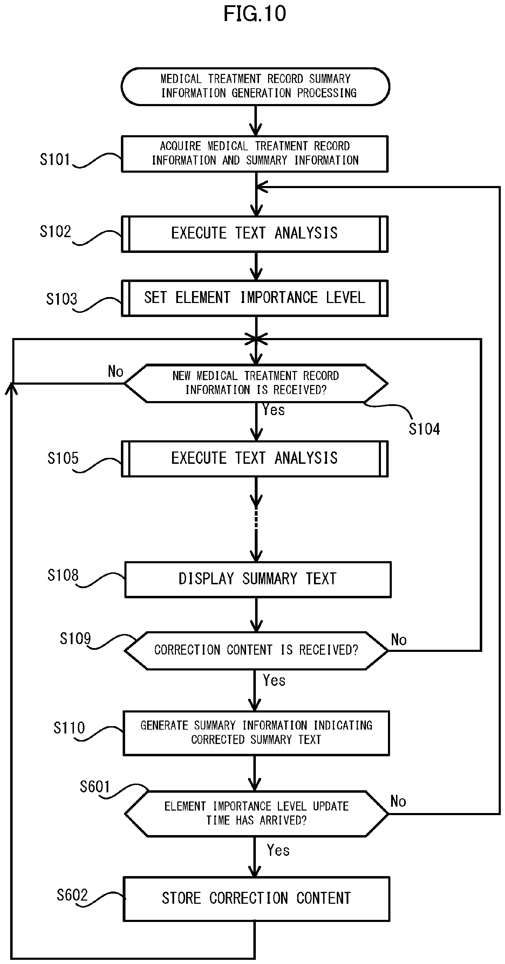

The medical treatment record summary information generation processing executed by the medical treatment record summary information generation device 1 according to this modified example will be described with reference to FIG. 10. In FIG. 10, processing similar to that in the embodiment is given the same sign as that in FIG. 4. First, after a series of processing operations from Steps S101 to S109 is executed, the summary generator 115 generates summary information indicating a corrected summary text, based on information indicating the correction content (Step S110). Next, the text analyzer 111 and the medical information analyzer 112 determine whether the element importance level update time has arrived (Step S601). When the text analyzer 111 and the medical information analyzer 112 determine that the element importance level update time has not arrived (Step S601: No), the processing in and after Step S102 is executed again. On the other hand, it is assumed that the text analyzer 111 and the medical information analyzer 112 determine that the element importance level update time has arrived (Step S601: Yes). In this case, the correction receiver 117 causes a correction content storage (unillustrated) provided in the auxiliary storage 13 to store the received correction content (Step S602). Subsequently, the processing in Step S104 is executed again.

Thus, in the medical treatment record summary information generation device 1 according to this modified example, every time an element importance level update time arrives, an element importance level is updated based on a correction content for a summary text received by the correction receiver 117 by then. Further, every time an element importance level update time arrives, a method of determining an element constituting a medical treatment record text may be updated based on a correction content for a summary text received by the correction receiver 117 by then, in the modified example described by use of FIG. 9 and FIG. 10.

This configuration can reduce execution frequency of the text analysis processing and the element importance level setting processing, or the medical treatment record text constituent determination processing using every piece of past medical treatment record information and summary information therefor and therefore can lighten processing load.

An example of the medical treatment record summary information generation device 1 generating summary information relating to medical treatment record information from the medical treatment record information has been described in the embodiment. However, without being limited to the above, for example, the medical treatment record summary information generation device 1 may generate summary information relating to nursing record information or care record information from the nursing record information or the care record information.

Summary information according to the embodiment may indicate a summary text translated into a plain text, based on an element with a high importance level extracted from medical treatment record information including a medical technical term. The "plain text" refers to a text understandable without knowledge of medical technical terms. In this case, for example, the medical language DB 134 may store each medical technical term in association with a plain expression relating thereto. Then, referring to a plain expression stored in the medical language DB 134, the summary generator 115 may generate a summary text translated into a plain text, based on an element with a high importance level estimated by the employed element estimator 114 from medical treatment record information including a medical technical term. This configuration provides an advantage that information included in a summary text can be shared among, for example, a doctor, a paramedic, a person involved in care, and a patient.

Further, various functions of the medical treatment record summary information generation device 1 according to the present disclosure can be provided by use of an ordinary computer system instead of a dedicated system. For example, the medical treatment record summary information generation device 1 executing the aforementioned processing may be configured by distributing a non-transitory computer-system-readable recording medium (such as a compact disc read only memory [CD-ROM]) storing a program for executing the operations described above to a computer connected to a network and installing the program on the computer system.

A program may be provided for a computer by any method. For example, a program may be uploaded to a bulletin board system (BBS) on a communication line and be distributed to a computer through the communication line. Then, the computer launches the program and executes the program similarly to other applications under control of an operating system (OS). Consequently, the computer functions as the medical treatment record summary information generation device 1 executing the aforementioned processing.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2018-165027, filed on September 4, 2018, and Japanese Patent Application No. 2019-048413, filed on March 15, 2019, of which the entirety of the disclosures is incorporated by reference herein.

### Industrial Applicability

The present disclosure is suitable for work of generating summary information from medical treatment record information on the front lines of health care.

### Reference Signs List

- 1: Medical treatment record summary information generation device
- 11: CPU
- 12: Main storage
- 13: Auxiliary storage
- 15: Input device
- 16: Display
- 19: Bus
- 111: Text analyzer
- 112: Medical information analyzer
- 113: Medical treatment record receiver
- 114: Employed element estimator
- 115: Summary generator
- 116: Summary output device
- 117: Correction receiver
- 131: Medical treatment record DB
- 132: Summary DB
- 133: Parameter DB
- 134: Medical language DB
- 136: Allowable range DB
- 137: Template DB

## Claims

1. A medical treatment record summary information generation device comprising:
a medical treatment record storage storing medical treatment record information;
a summary storage storing summary information indicating a summary text relating to the medical treatment record information in association with the medical treatment record information;
an importance level storage storing importance level information indicating an importance level of each element constituting a medical treatment record text indicated by the medical treatment record information and being acquired by performing text analysis on the medical treatment record text, the importance level information being generated based on the element and a summary text relating to the medical treatment record text;
a medical treatment record receiver receiving new medical treatment record information;
an employed element estimator estimating an element constituting a summary text, based on the importance level information and an element constituting a medical treatment record text indicated by new medical treatment record information received by the medical treatment record receiver and being acquired by performing text analysis on the medical treatment record text;
a summary generator generating a summary text by use of an element estimated by the employed element estimator; and
a correction receiver receiving a correction content for the summary text by a user,
wherein the employed element estimator generates new importance level information, based on a summary text after being corrected along a correction content received by the correction receiver and an element constituting the medical treatment record text and updates importance level information stored by the importance level storage with generated new importance level information,

2. The medical treatment record summary information generation device according to claim 1, further comprising:
a medical linguistic information storage storing medical linguistic information indicating a medical language; and
a medical information analyzer extracting, with reference to the medical linguistic information, an element relating to a medical-field-specific expression from an element constituting a medical treatment record text and being acquired by performing the text analysis and by executing semantic analysis, estimating a medical meaning including a meaning of whether an inspection value is normal or whether a variation in an inspection value is normal, for each extracted element.

3. The medical treatment record summary information generation device according to claim 1 or 2, further comprising
a text analyzer estimating an element constituting the medical treatment record text by determining at least one of a method of punctuating a sentence in the summary text, a method of summary text structuring, and a form of expressing the summary text.

4. The medical treatment record summary information generation device according to any one of claims 1 to 3, further comprising:
an important element storage storing important element information indicating a predetermined important element; and
an important element output device outputting the important element information stored by the important element storage.

5. A method of generating medical treatment record summary information, the method comprising:
a step of receiving new medical treatment record information;
a step of estimating an element constituting a summary text, based on importance level information stored by an importance level storage and an element constituting a medical treatment record text indicated by received new medical treatment record information and being acquired by performing text analysis on the medical treatment record text;
a step of generating a summary text by use of an estimated element;
a step of receiving a correction content for the summary text by a user; and
a step of generating new importance level information, based on a summary text after being corrected along the correction content and an element constituting a medical treatment record text indicated by new medical treatment record information and being acquired by performing text analysis on the medical treatment record text and updating importance level information stored by the importance level storage with generated new importance level information.

6. A program causing a computer to function as:
a medical treatment record storage storing medical treatment record information;
a summary storage storing summary information indicating a summary text relating to the medical treatment record information in association with the medical treatment record information;
an importance level storage storing importance level information indicating an importance level of each element constituting a medical treatment record text indicated by the medical treatment record information and being acquired by performing text analysis on the medical treatment record text, the importance level information being generated based on the element and a summary text relating to the medical treatment record text;
a medical treatment record receiver receiving new medical treatment record information;
an employed element estimator estimating an element constituting a summary text, based on the importance level information and an element constituting a medical treatment record text indicated by new medical treatment record information received by the medical treatment record receiver and being acquired by performing text analysis on the medical treatment record text;
a summary generator generating a summary text by use of an element estimated by the employed element estimator; and
a correction receiver receiving a correction content for the summary text by a user,
wherein the employed element estimator generates new importance level information, based on a summary text after being corrected along a correction content received by the correction receiver and an element constituting the medical treatment record text and updates importance level information stored by the importance level storage with generated new importance level information.
